# EUROPÄISCHE PATENTSCHRIFT

(11) **EP 1 007 510 B1**
(45) Veröffentlichungstag und Bekanntmachung des Hinweises auf die Patenterteilung: **27.03.2002**
(21) Anmeldenummer: 98948890.3
(22) Anmeldetag: 28.08.1998
(51) Int. Cl.: C07C 329/02

(54) **VERFAHREN ZUR HERSTELLUNG VON THIOCHLORFORMIATEN**
METHOD FOR PRODUCING THIOCHLOROFORMATES
PROCEDE DE PRODUCTION DE THIOCHLOROFORMIATES

(30) Priorität: 28.08.1997 DE 19737619
(43) Veröffentlichungstag der Anmeldung: 14.06.2000
(73) Patentinhaber: BASF AKTIENGESELLSCHAFT, 67056 Ludwigshafen (DE)
(72) Erfinder: WEYER, Hans-Jürgen, D-67240 Bobenheim-Roxheim (DE); STAMM, Armin, D-55128 Mainz (DE); WEBER, Theodor, D-67063 Ludwigshafen (DE); HENKELMANN, Jochem, D-68165 Mannheim (DE)
(74) Vertreter: Isenbruck, Günter, Dr.
(86) Internationale Anmeldenummer: EP9805525
(87) Internationale Veröffentlichungsnummer: WO9911611

(56) Entgegenhaltungen:
- EP-A- 0 024 683
- EP-A- 0 053 981
- EP-A- 0 309 844
- US-A- 3 277 143

## Beschreibung

Die Erfindung betrifft ein Verfahren zur Herstellung von Thiochlorformiaten durch Umsetzung von Thiolen mit Phosgen in Gegenwart eines Katalysators.

Thiochlorformiate sind bedeutende Zwischenprodukte in der organischen Synthese insbesondere von physiologischen Wirkstoffen wie Arznei- und Pflanzenschutzmitteln. Thiochlorformiate werden in einem seit langem bekannten Verfahren durch Umsetzung von Thiolen mit Phosgen erhalten. Da die Reaktion von Thiolen mit Phosgen sehr langsam und in erheblichem Umfang unter Bildung von Nebenprodukten wie Thiocarbonaten und Disulfiden abläuft, wird sie üblicherweise in Gegenwart von Katalysatoren durchgeführt.

Aus der US 3,299,114 ist die Herstellung von Thiochlorformiaten durch Phosgenierung der entsprechenden Thiole (Mercaptane) bekannt, wobei als Katalysatoren offenkettige oder heterocyclische tertiäre Amine oder heterocyclische aromatische Stickstoffbasen vom Typ des Pyridins verwendet werden. Die hierbei erzielten Ausbeuten an Thiochlorformiaten lassen jedoch zu wünschen übrig.

In EP-A 0 024 683 und EP-A 0 053 981 werden Carbonsäureamide und acyclische Harnstoffe als Katalysatoren vorgeschlagen. Nachteilig an diesen Verfahren sind die immer noch unbefriedigenden Ausbeuten, der hohe Anteil an Nebenprodukten sowie der hohe Katalysatorgehalt der Synthesegemische. Die gebildeten Thiochlorformiate müssen deshalb anschließend in einem weiteren Verfahrensschritt, in der Regel durch Destillation, aufgearbeitet werden.

In der DE-A 41 37 012 sind organische Phosphorverbindungen als Katalysatoren für die Herstellung von Thiochlorformiaten offenbart. Nachteilig an diesen Verfahren ist der Anfall phosphorhaltiger Destillationsrückstände, deren Entsorgung wegen der Bildung von Phosphorsäuren bei der Verbrennung erschwert ist.

Der Erfindung liegt die Aufgabe zugrunde, den zuvor genannten Nachteilen abzuhelfen, insbesondere ein Verfahren zur Herstellung von Thiochlorformiaten bereitzustellen, das mit sehr geringen Katalysatormengen auskommt und die Bildung von Nebenprodukten weitgehend vermeidet.

Die Aufgabe wird gelöst durch ein Verfahren zur Herstellung von Thiochlorformiaten aus Thiolen durch Umsetzung mit Phosgen (Phosgenierung) in Gegenwart eines Katalysators, das dadurch gekennzeichnet ist, daß als Katalysator ein cyclischer Harnstoff oder Thioharnstoff verwendet wird. Unter cyclischen Harnstoffen oder Thioharnstoffen sollen solche Verbindungen verstanden werden, in denen die Harnstoff- bzw. Thioharnstoffgruppe Teil eines Ringsystems ist. Der cyclische Harnstoff oder Thioharnstoff kann sowohl als solcher als auch in Form seiner durch Umsetzung mit Halogenwasserstoffsäuren oder Phosgen erhältlichen Salze vorliegen.

Cyclische Harnstoffe oder Thioharnstoffe sind an sich bekannt. Ihre Herstellung ist beispielsweise in Houben-Weyl, Methoden der organischen Chemie, Band E4, 4. Auflage 1983 beschrieben.

Als Katalysatoren kommen insbesondere cyclische Harnstoffe oder Thioharnstoffe der allgemeinen Formel I in Frage: 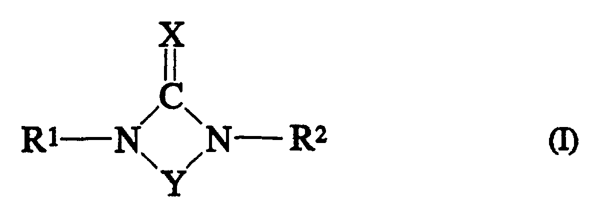 worin
X ein Sauerstoff- oder Schwefelatom bedeutet,
Y eine, gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und Cyano ein- bis dreifach substituierte gesättigte oder ein- oder mehrfach olefinisch ungesättigte Kohlenstoffkette mit 2 bis 8 C-Atomen, die eine Ether-, Thioether-, tertiäre Amino-, Keto-, Lacton-, alkylsubstituierte Lactam-, Sulfon- oder Diketogruppe enthalten kann, oder eine, gegebenenfalls mit C₁-C₄-Alkyl, C₁-C₄-Alkoxy, Phenoxy, C₂-C₄-Acyl, C₂-C₄-Acyloxy, C₂-C₈-Dialkylamino, Halogen, Nitro und /oder Cyano ein- bis dreifach substituierte Cycloalkylen mit 5 bis 12 C-Atomen, Heterocycloalkylengruppe mit 4 bis 11 C-Atomen, Arylengruppe mit 6 bis 12 C-Atomen oder Heteroarylengruppe mit 3 bis 11 C-Atomen bedeutet und
R¹, R², die gleich oder verschieden sein können,
   gegebenenfalls mit C₁-C₄-Alkoxy, C₂-C₄-Acyl, C₂-C₄-Acyloxy, C₂-C₈-Dialkylamino, Halogen, Nitro und/oder Cyano ein- bis dreifach substituiertes C₁-C₂₀-Alkyl, C₂-C₂₀-Alkenyl oder C₂-C₂₀-Alkinyl, C₂-C₂₀-Acyl oder gegebenenfalls mit C₁-C₄-Alkyl, C₁-C₄-Alkoxy, C₂-C₄-Acyl, C₂-C₄-Acyloxy, C₂-C₈-Dialkylamino, Halogen, Nitro und/oder Cyano ein- bis dreifach substituiertes C₃-C₈-Cycloalkyl, C₄-C₂₀-Cycloalkyl-alkyl, C₃-C₈-Heterocycloalkyl, C₄-C₂₀-Heterocycloalkyl-alkyl, C₆-C₁₄-Aryl oder C₇-C₂₀-Arylalkyl bedeuten oder R¹ und R² Teil einer beide Stickstoffatome verbindenden einfach oder mehrfach olefinisch ungesättigten Kohlenstoffkette mit 2 bis 8 C-Atomen, Cycloalkylengruppe mit 5 bis 12 C-Atomen, Heterocyloalkylengruppe mit 4 bis 11 C-Atomen, Arylengruppe mit 6 bis 12 C-Atomen oder Heteroarylengruppe mit 3 bis 11 C-Atomen sind.

Y steht für eine gesättigte oder ein- oder mehrfach ungesättigte Kohlenstoffkette mit 2 bis 8 C-Atomen wie C₂-C₈-Alkylen, C₂-C₈-Alkenylen, C₄-C₈-Alkadienylen oder C₆-C₈-Alkatrienylen. Beispiele sind Ethylen, Propylen, Butylen, Pentylen, Hexylen, Heptylen, Octylen, Vinylen, Propenylen, 1-Butenylen, 2-Butenylen, Butadienylen, 1-Pentenylen, 1,3-Pentadienylen, 1,4-Pentadienylen, 1-Hexenylen, 1,3-Hexadienylen und 1,3,5-Hexatrienylen, bevorzugt Ethylen, Propylen und Vinylen. Die Kohlenstoffkette kann auch gegenüber Phosgen und Thiol unter den Reaktionsbedingungen inerte Gruppen wie eine Ether-, Thioether-, tertiäre Amino-, Keto-, Lacton-, substituierte Lactam-, Sulfon- oder eine α-Diketogruppe enthalten, wobei im Falle einer C₂-Kette Y auch ausschließlich aus einer α-Diketogruppe bestehen kann.

Y kann weiterhin eine (divalente) Cycloalkylengruppe mit 5 bis 12 C-Atomen bedeuten, die über vicinale C-Atome oder in 1,3-Stellung gebunden sein kann. Beispiele sind 1,2- oder 1,3-Cyclopentylen, 1,2- oder 1,3-Cyclohexylen und 1,2- oder 1,3-Cycloheptylen. Die Cycloalkylengruppe kann an Stelle von einem oder mehreren C-Atomen auch ein oder mehrere Heteroatome wie Stickstoff, Sauerstoff oder Schwefel enthalten. Y kann ferner eine (divalente) Arylengruppe mit 6 bis 12 C-Atomen bedeuten, die über vicinale C-Atome gebunden ist. Beispiele sind ortho-Phenylen, 1,2- oder 2,3-Naphthylen, und 1,2 oder 2,3-Anthracenylen, bevorzugt ortho-Phenylen. Die Arylengruppe kann an Stelle von einem oder mehreren C-Atomen auch ein oder mehrere Heteroatome wie Stickstoff enthalten. Beispiele sind 2,3- oder 3,4-Pyrrolylen, 2,3- oder 3,4-Pyridinylen, 2,3-, 3,4-, 5,6 oder 6,7-Chinolinylen- oder 2,3-, 5,6- oder 6,7-Chinoxalinylen.

Die fir Y vorstehend genannten Gruppen können ferner mit unter den Reaktionsbedingungen gegenüber Phosgen und Thiol inerten Gruppen wie C₁-C₄-Alkyl, C₁-C₄-Alkoxy, C₂-C₄-Acyl, C₁-C₄-Acyloxy, Phenoxy, C₂-C₈-Dialkylamino, Halogen, Nitro und Cyano ein- bis dreifach substituiert sein.

R¹, R² können bedeuten:
- C₁-C₂₀-Alkyl, bevorzugt C₁-C₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁-C₄-Alkyl wie Methyl, Ethyl, n-Propyl, iso-Propyl, n-Butyl, iso-Butyl, sec.-Butyl und tert.-Butyl, speziell Methyl.
- C₂-C₂₀-Alkenyl, bevorzugt C₂-C₈-Alkenyl, wie Vinyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1-Ethyl-1-propenyl, 1-Ethyl-2-propenyl, 1-Hexenyl, 2-Hexenyl, 3-Hexenyl, 4-Hexenyl, 5-Hexenyl, besonders bevorzugt C₂-C₄-Alkenyl, speziell Vinyl.
- C₂-C₂₀-Alkinyl, bevorzugt C₂-C₈-Alkinyl, wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, besonders bevorzugt C₂-C₄-Alkinyl, speziell Propinyl.
- C₂-C₂₀-Acyl, bevorzugt C₂-C₈-Acyl, besonders bevorzugt C₂-C₄-Acyl wie Acetyl, Propionyl, Butyryl und iso-Butyryl,

Die für R¹, R² vorstehend genannten Gruppen können mit C₁-C₄-Alkoxy, C₂-C₄-Acyl, C₂-C₄-Acyloxy, C₂-C₈-Dialkylamino, Halogen, Nitro und/oder Cyano substituiert sein. Bevorzugte substituierte Reste sind substituierte Alkylreste, besonders bevorzugt mit Halogen, Cyano und Alkoxy substiutierte Alkylreste wie Cyanmethyl, Chlormethyl und Methoxymethyl.

R¹, R² stehen weiterhin für
- C₃-C₈-Cycloalkyl, bevorzugt C₅-C₇-Cycloalkyl wie Cyclopentyl, Cyclohexyl und Cycloheptyl, besonders bevorzugt Cyclohexyl,
- C₄-C₂₀-Cycloalkyl-alkyl, bevorzugt C₄-C₁₂-Cycloalkyl-alkyl, wie Cyclopropyl-methyl, Cyclobutyl-methyl, Cyclopentyl-methyl, Cyclohexyl-methyl, Cyclopropyl-ethyl, Cyclobutyl-ethyl, Cyclopentyl-ethyl, Cyclohexyl-ethyl, Cyclopropyl-propyl, Cyclobutyl-propyl, Cyclopentyl-propyl, Cyclohexyl-propyl,
- C₃-C₈-Heterocycloalkyl-, bevorzugt ein 5- oder 6-gliedriger Ring mit einem oder zwei O-, N- und/oder S-Atomen im Ring, der aromatisch oder nichtaromatisch sein kann, wie 2- oder 3-Furyl, 2- oder 3-Thienyl, 2- oder 3-Pyrrolyl, 2- oder 4-Imidazolyl, 2- oder 3-Oxazolyl, 2- oder 3-Thiazolyl, Pyridinyl, Morpholyl, Thiomorpholyl und Pyrazolyl,
- C₄-C₂₀-Heterocycloalkyl-alkyl,
- C₆-C₁₄-Aryl, wie Phenyl, 1-Naphthyl, 2-Naphthyl, 1-Anthryl, 2-Anthryl, 9-Anthryl, bevorzugt Phenyl, 1-Naphtyl, 2-Naphtyl, besonders bevorzugt Phenyl,
- C₇-C₂₀-Arylalkyl, bevorzugt C₇-C₁₂-Phenylalkyl, wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl.

Die genannte Gruppen können mit C₁-C₄-Alkyl, C₁-C₄-Alkoxy, C₂-C₄-Acyl, C₂-C₄-Acyloxy, C₂-C₈-Dialkylamino, Halogen, Nitro und/oder Cyano substituiert sein.

Unter den genannten Resten R¹ und R² sind Arylreste wie Phenyl, unsubstituierte Alkyl- und Acylreste wie Methyl und Acetyl, sowie mit Halogen oder Cyano substituierte Alkylreste wie Chlormethyl und Cyanmethyl besonders bevorzugt.

R¹ und R² können ferner Teil einer beide Stickstoffatome verbindenden gesättigten oder ein- oder mehrfach ungesättigten Kohlenstoffkette mit 2 bis 8 C-Atomen, einer Cycloalkylengruppe mit 5 bis 12 C-Atomen, Heterocycloalkylengruppe mit 4 bis 11 C-Atomen, einer Arylengruppe mit 6 bis 12 C-Atomen oder einer Heteroarylengruppe mit 3 bis 11 C-Atomen sein. Es kommen die vorstehend für Y genannten Alkylen-, Alkenylen-, Alkadienylen-, Alkatrienylen-, Cylo- bzw. Heterocycloalkylen- und Arylen- bzw. Heteroarylengruppen in Frage. Bevorzugt sind Ethylen, Vinylen und Phenylen.

Ferner kommen als Katalysatoren Verbindungen der allgemeinen Formel II in Frage: 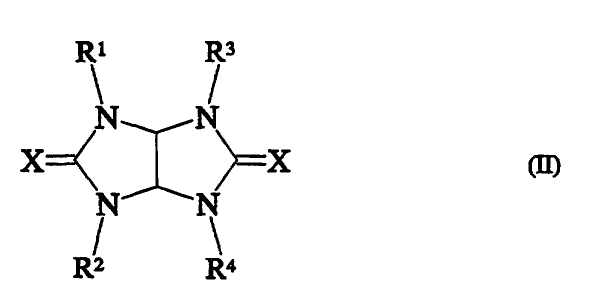 worin X, R¹ und R² die oben angegebenen Bedeutungen und R³ und R⁴ die Bedeutungen von R¹ und R² haben,
oder Verbindungen der allgemeinen Formel III, 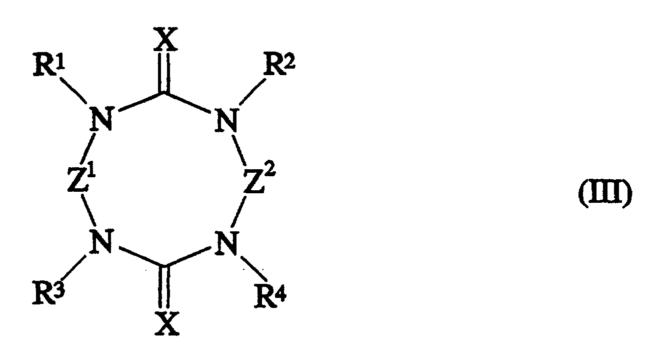 worin X, R¹ bis R⁴ die oben angegebenen Bedeutungen haben und Z¹, Z², die gleich oder verschieden sein können, gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und/oder Cyano ein- bis dreifach substituierte Methylen-, Ethylen- oder Vinylengruppen bedeuten. Bevorzugt ist Methylen.

Nachstehend seien in beispielhafter Weise einige besonders geeignete Katalysatoren aufgeführt:
- N,N'-Dimethylethylenharnstoff
- N,N'-Dibutylethylenthioharnstoff
- N,N'-Diacetylethylenharnstoff
- N,N'-Dimethylpropylenharnstoff
- N-Chlormethyl-N'-cyanomethylpropylenharnstoff
- N-Methyl-N'-ethylpropylenharnstoff
- 1,3-Dimethyl-1,3-dihydrobenzimidazol-2-on
- 1-Methyl-3-phenylimidazolidin-2,4,5-trion
- 1,3,4,6-Tetramethyl-1,3,4,6-tetrahydro-imidazo[4,5-d]imidazol-2,5-dion
- 4-Methoxy-1-methyl-3-phenylimidazolidin-2-thion

Als Katalysatoren insbesondere bevorzugt sind N,N'-Dimethylethylenharnstoff und N,N'-Dimethylpropylenharnstoff.

Die genannten cyclischen Harnstoffe und Thioharnstoffe können als solche, in Form ihrer Salze mit Halogenwasserstoffsäuren, beispielsweise als Hydrochloride, oder in Form ihrer durch Umsetzung mit Phosgen erhältlichen Salze (Vilsmeier-Salze) eingesetzt werden.

Als Thiole können in dem erfindungsgemäßen Verfahren Thiole mit einer oder mehreren SH-Gruppen eingesetzt werden. Repräsentativ sind die in EP-A 0 024 683 und in US 3,299,114 genannten Thiole. Besonders bevorzugt sind C₁-C₈-Alkanthiole wie Ethanthiol, Ethandithiol, Butanthiol und Octanthiol.

Das erfindungsgemäße Verfahren kann als homogen katalysierte Flüssigphase-Reaktion durchgeführt werden. Als flüssiges Reaktionsmedium kommen das einzusetzende Thiol, das davon abgeleitetes Thiochlorformiat oder ein inertes Lösungsmittel oder deren Mischungen in Frage.

Das erfindungsgemäße Verfahren kann in der Schmelze des einzusetzenden Thiols durchgeführt werden. Dazu wird das Thiol mit dem Katalysator vorgelegt und Phosgen eingeleitet. Beim Arbeiten mit bei Raumtemperatur festen Thiolen kann es vorteilhaft sein, das Thiol zunächst durch Erwärmen in flüssige Form zu bringen und anschließend Phosgen einzuleiten. Es kann in Fällen, in denen es nach Anspringen der Reaktion durch die Reaktionswärme der exothermen Reaktion zur Verflüssigung der Komponenten kommt, auch ausreichend sein, das Thiol und den Katalysator in fester Form vorzulegen und Phosgen einzuleiten. Die Erwärmung des Reaktionsgemisches auf die angestrebte Reaktionstemperatur kann durch äußere Wärmezufuhr oder ohne äußere Wärmezufuhr unter Ausnutzung der Reaktionswärme geschehen. Vorzugsweise werden Thiol und Katalysator in flüssiger Form vorgelegt.

Das erfindungsgemäße Verfahren kann auch in einem Lösungsmittel durchgeführt werden. Als Lösungsmittel eignet sich insbesondere das von dem einzusetzenden Thiol abgeleitete Thiochlorformiat. Es kann jedoch auch in einem zusätzlichen, gegenüber Thiol und Phosgen inerten Lösungsmittel, das ein ausreichendes Lösungsvermögen für die Reaktanten und den Katalysator aufweist, gearbeitet werden. Das zusätzliche inerte Lösungsmittel kann allein oder in Mischung mit dem Thichlorformiat eingesetzt werden. Das Arbeiten in einem zusätzlichen inerten Lösungsmittel kann vor allem dann vorteilhaft sein, wenn der Schmelzpunkt des Thiols oder des davon abgeleiteten Thiochlorformiats oberhalb der angestrebten Reaktionstemperatur liegen. Auch kann die Verwendung eines zusätzlichen inerten Lösungsmittels der Abführung der Reaktionswärme dienen. Als zusätzliche inerte Lösungsmittel eignen sich aliphatische und aromatische Kohlenwasserstoffe wie Pentan, Hexan, Cyclohexan, Toluol, Xylol oder Benzol, halogenierte Kohlenwasserstoffe wie Trichlorethan, Chlorbenzol oder Dichlorbenzol oder Ester wie Ethylacetat oder Butylacetat.

Bevorzugt wird das erfindungsgemäße Verfahren in der Schmelze des einzusetzenden Thiols oder in dem davon abgeleiteten Thiochlorformiat als Lösungsmittel ohne Verwendung eines zusätzlichen inerten Lösungsmittels durchgeführt.

Das erfindungsgemäße Verfahren kann kontinuierlich oder diskontinuierlich durchgeführt werden. Bevorzugt wird das erfindungsgemäße Verfahren kontinuierlich durchgeführt, beispielsweise in einem Rührkessel, in einer Rührkesselkaskade, in einem Schlaufenreaktor oder in einer Gegenstromkolonne. Die Umsetzungstemperatur beträgt im allgemeinen -20 bis 180°C, bevorzugt 0 bis 120°C, besonders bevorzugt von 20 bis 80°C, speziell 45 bis 65 °C. Die Umsetzung wird im allgemeinen bei Drücken von 0,01 bis 50 bar, bevorzugt bei 0,5 bis 5 bar, besonders bevorzugt bei Atmosphärendruck durchgeführt.

Der Katalysator wird im allgemeinen, bezogen auf die Menge des eingesetzten Thiols, in Mengen von 0,01 bis 20 Mol.-% eingesetzt. Die Menge des Katalysators richtet sich auch danach, ob die Umsetzung in dem einzusetzenden Thiol allein oder in Gegenwart von Lösungsmittel durchgeführt wird. Die verwendete Katalysatormenge liegt oft so niedrig, daß eine Abtrennung des Katalysators von dem Reaktionsprodukt entfallen kann. Sie liegt bevorzugt zwischen 0,02 und 1 Mol-%, besonders bevorzugt zwischen 0,02 und 0,1 Mol-%. Besonders niedrige Katalysatormengen können bei Umsetzung in der Schmelze des Thiols ohne Verwendung von Lösungsmittel eingesetzt werden. So kann die verwendet Katalysatormenge unterhalb 0,05 Mol-% liegen.

Das Molverhältnis von Phosgen zum Thiol beträgt im allgemeinen 0,5:1 bis 50:1. Üblicherweise wird man mit einem Überschuß an Phosgen arbeiten, da anderenfalls unumgesetztes Thiol zurückbliebe. Ferner begünstigt ein Überschuß an Thiol Nebenreaktionen wie die Bildung von Dithiocarbonaten aus Thiol und bereits gebildetem Thiochlorformiat. Andererseits bringt ein hoher Phosgen-Überschuß (beispielsweise größer als 2:1) keinen weiteren Vorteil. Das Molverhältnis von Phosgen zu Thiol beträgt daher bevorzugt 1:1 bis 2:1, besonders bevorzugt 1:1 bis 1,5:1, insbesondere 1:1 bis 1,2:1.

Mit dem erfindungsgemäßen Verfahren können sehr hohe Thiochlorformiat-Ausbeuten erhalten werden. So beträgt die Thiochlorformiat-Ausbeute, bezogen auf eingesetztes Thiol, im allgemeinen mehr als 98 Mol-%, bevorzugt mehr als 99 Mol-%. Die Ausbeute kann mehr als 99,5 Mol-% betragen.

Der Phosgenierungsreaktion können sich ein oder mehreren Schritten zur Aufreinigung des Reaktionsproduktes anschließen. So kann gegebenenfalls der flüssige Reaktionsaustrag der Phosgenierungsreaktion durch einen mechanische Abtrennung wie beispielsweise eine Klärfiltration von unlöslichen Verunreinigungen befreit werden. Oft ist ein solcher mechanischer Abtrennschritt schon ausreichend, um ein Produkt ausreichend hoher Reinheit zu erhalten, und eine weitere Aufarbeitung kann entfallen. Es können sich jedoch weitere Aufreinigungsschritte zur Abtrennung löslicher Verunreinigungen, beispielsweise durch Destillation oder Umkristallisieren, anschließen.

Das gebildete Thiochlorformiat zeichnet sich durch eine sehr hohe Reinheit aus. Die Reinheit des Rohproduktes, beträgt im allgemeinen > 98 Mol-%, bevorzugt > 99 Mol-%. Es können Reinheiten von > 99,5 Mol-% erhalten werden. Unter dem Rohprodukt soll das Reaktionsgemisch nach Durchführung der Umsetzung, gegebenenfalls nach Durchführung einer mechanischen Abtrennung, aber vor der Durchführung weiterer Aufreinigungsschritte zur Entfernung löslicher Verunreinigungen, verstanden werden. Unter der Reinheit soll der Thiochlorformiatgehalt des Reaktionsgemisches verstanden werden, der bei Verwendung eines Lösungsmittels nur auf den Teil des Reaktionsgemisches bezogen ist, der zu Beginn der Umsetzung nicht Lösungsmittel war.

Als Nebenbestandteil kann das Rohprodukt den Katalysator, Nebenprodukte sowie unumgesetztes Thiol enthalten. Nebenprodukten sind besipielsweise Dithiocarbonate und Disulfide. Im allgemeinen beträgt die Menge an Dithiocarbonat im Rohprodukt < 1 Mol-%, bevorzugt < 0,5 Mol-%, besonders bevorzugt < 0,1 Mol-%. Die Menge an Disulfid im Rohprodukt beträgt im allgemeinen < 0,5 Mol-%, bevorzugt < 0,1 Mol-%, besonders bevorzugt < 0,01 Mol-%. Die Menge an unumgesetztem Thiol beträgt im allgemeinen < 0,1 Mol-%, bevorzugt < 0,01 Mol-%. Die %-Angaben beziehen sich bei Verwendung eines Lösungsmittels auf den Teil des Reaktionsgemisches, der zu Beginn der Umsetzung nicht Lösungsmittel war.

Vorteilhaft an dem erfindungsgemäßen Verfahren ist, daß die Umsetzung unter vergleichsweise milden Bedingungen und in Gegenwart sehr geringer Katalysatormengen weitgehend vollständig und mit hoher Selektivität verläuft. Die Reaktionsausträge können wegen des geringen Gehalts an Nebenbestandteilen farblos sein. Dadurch kann eine Aufarbeitung des Rohproduktes entfallen.

Die Erfindung wird durch die nachstehenden Beispiele näher erläutert.

### Beispiele 1 und 2 und Vergleichsbeispiele V 1 - V 4

In einer Mischung aus n-Octanthiol und X mol Katalysator im Molverhältnis 1:X wurde bei einer Temperatur von T °C im Laufe von t Stunden ein 1,1-facher Überschuß, bezogen auf Octanthiol, an Phosgen eingeleitet. Anschließend wurde das Gemisch noch eine Stunde bei der Reaktionstemperatur gehalten. Danach wurde überschüssiges Phosgen sowie restlicher Chlorwasserstoff mit Stickstoff aus dem Gemisch ausgeblasen. Das so erhaltene Rohprodukt wurde nach einer Klärfiltration gaschromatographisch untersucht. Vergleichsbeispiele V 1 und V 2 wurden gemäß DE-A 41 37 012, Vergleichsbeispiel V 3 gemäß EP-A 0 053 981 und Vergleichsbeispiel V 4 gemäß EP-A 0 024 683 durchgeführt.

Einzelheiten zu diesen Versuchen sind der nachstehenden Tabelle zu entnehmen. Mit dem erfindungsgemäßen Verfahren kann der Anteil an Nebenbestandteilen gegenüber den aus dem Stand der Technik bekannten Verfahren auf ein Fünftel reduziert werden.

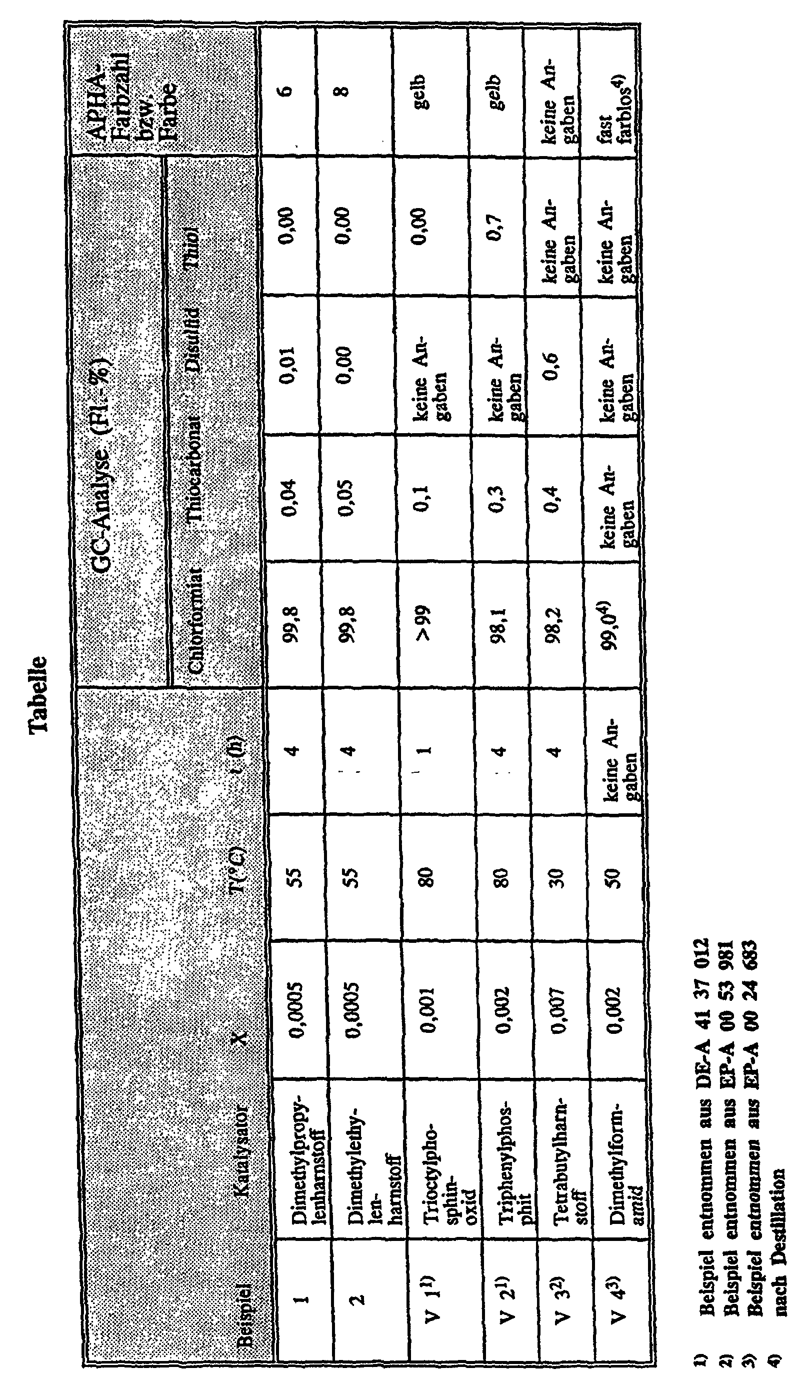

## Patentansprüche

1. Verfahren zur Herstellung von Thiochlorformiaten durch Umsetzung von Thiolen mit Phosgen in Gegenwart eines Katalysators, **dadurch gekennzeichnet, daß** man als Katalysator einen cyclischen Harnstoff oder Thioharnstoff verwendet, der in Form seiner durch Umsetzung mit Halogenwasserstoffsäuren oder Phosgen erhältlichen Salze vorliegen kann.

2. Verfahren nach Anspruch 1, **dadurch gekennzeichnet, daß** man als Katalysator eine Verbindung der allgemeinen Formel I 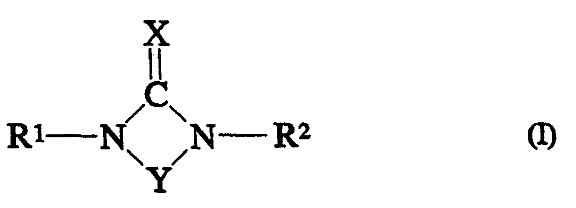 worin
X ein Sauerstoff- oder Schwefelatom bedeutet,
Y eine, gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und Cyano ein- bis dreifach substituierte gesättigte oder ein- oder mehrfach olefinisch ungesättigte Kohlenstoffkette mit 2 bis 8 C-Atomen, die eine Ether-, Thioether-, tertiäre Amino-, Keto-, Lacton-, alkylsubstituierte Lactam-, Sulfon- oder Diketogruppe enthalten kann, oder eine, gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und Cyano ein- bis dreifach substituierte Cycloalkylengruppe mit 5 bis 12 C-Atomen, Heterocycloalkylengruppe mit 4 bis 11 C-Atomen, Arylengruppe mit 6 bis 12 C-Atomen oder Heteroarylengruppe mit 3 bis 11 C-Atomen bedeutet
und
R¹, R², die gleich oder verschieden sein können,
gegebenenfalls mit C₁-C₄-Alkoxy, C₂-C₄-Acyl, C₂-C₄-Acyloxy, C₂-C₈-Dialkylamino, Halogen, Nitro und/oder Cyano ein- bis dreifach substituiertes C₁-C₂₀-Alkyl, C₂-C₂₀-Alkenyl, C₂-C₂₀-Alkinyl oder C₂-C₂₀-Acyl, oder gegebenenfalls mit C₁-C₄-Alkyl, C₁-C₄-Alkoxy, C₂-C₄-Acyl, C₂-C₄-Acyloxy, C₂-C₈-Dialkylamino, Halogen, Nitro und/oder Cyano ein- bis dreifach substituiertes C₃-C₈-Cycloalkyl, C₄-C₂₀-Cycloalkyl-alkyl, C₃-C₈-Heterocycloalkyl, C₄-C₂₀-Heterocycloalkyl-alkyl, C₆-C₁₄-Aryl oder C₇-C₂₀-Arylalkyl bedeuten
oder
R¹ und R² Teil einer beide Stickstoffatome verbindenden gesättigten oder ein- oder mehrfach olefinisch ungesättigten Kohlenstoffkette mit 2 bis 8 C-Atomen, Cycloalkylengruppe mit 5 bis 12 C-Atomen, Heterocycloalkylengruppe mit 4 bis 11 C-Atomen, Arylengruppe mit 6 bis 12 C-Atomen oder Heteroarylengruppe mit 3 bis 11 C-Atomen sind,
und/oder eine Verbindung der allgemeinen Formel II 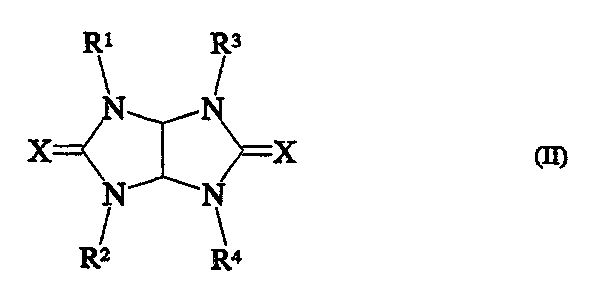 worin X, R¹ und R² die oben angegebenen Bedeutungen und R³ und R⁴ die Bedeutungen von R¹ und R² haben,
oder
eine Verbindung der allgemeinen Formel III, 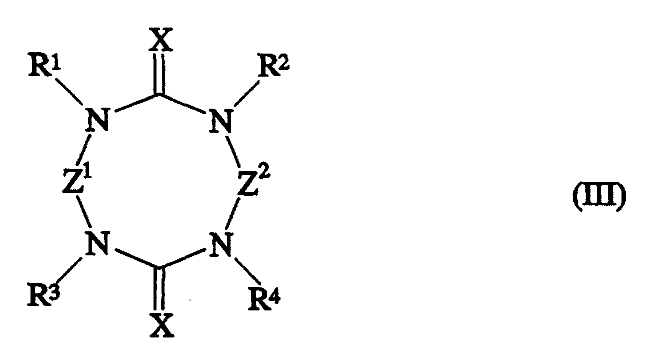 worin X, R¹ bis R⁴ die oben angegebenen Bedeutungen und Z¹, Z², die gleich oder verschieden sein können, eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und/oder Cyano ein- bis dreifach substituierte Methylen-, Ethylen- oder Vinylengruppe bedeuten, verwendet.

3. Verfahren nach Anspruch 1 oder 2, **dadurch gekennzeichnet, daß** man als Katalysator N,N'-Dimethylpropylen- oder N,N'-Dimethylethylenharnstoff verwendet.

4. Verfahren nach einem der Ansprüche 1 bis 3, **dadurch gekennzeichnet, daß** man C₁-C₈-Alkanthiole, C₅-C₈-Cycloalkanthiole, C₆- und C₁₂-Arylthiole, C₇-C₁₂-Aralkylthiole und/oder C₃-C₁₂-Heterocycloalkylthiole umsetzt.

5. Verfahren nach einem der Ansprüche 1 bis 4, **dadurch gekennzeichnet, daß** man pro Äquivalent Thiol 0,02 bis 1 Mol-% des Katalysators einsetzt.

6. Verfahren nach einem der Ansprüche 1 bis 5, **dadurch gekennzeichnet, daß** man die Umsetzung bei Temperaturen von 20 bis 80°C und Atmosphärendruck durchführt.

7. Verfahren nach einem der Ansprüche 1 bis 6, **dadurch gekennzeichnet, daß** man die Umsetzung in der Schmelze des Thiols durchführt.

8. Verfahren nach einem der Ansprüche 1 bis 7, **dadurch gekennzeichnet, daß** man die Umsetzung in Gegenwart eines Lösungsmittels durchführt.

9. Verfahren nach Anspruch 8, **dadurch gekennzeichnet, daß** man als Lösungsmittel das von dem eingesetzten Thiol abgeleitete Thiochlorformiat verwendet

10. Verwendung cyclischer Harnstoffe oder Thiobarnstoffe als Katalysatoren zur Herstellung von Thiochlorformiaten durch Umsetzung von Thiolen mit Phosgen in einem Verfahren gemäß Anspruch 1.

## Claims

1. Process for preparing thiochloroformates by reacting thiols with phosgene in the presence of a catalyst, wherein the catalyst used is a cyclic urea or thiourea which may be in the form of its salts obtainable by reaction with hydrohalic acids or phosgene.

2. Process as claimed in claim 1, wherein the catalyst used is a compound of the formula I 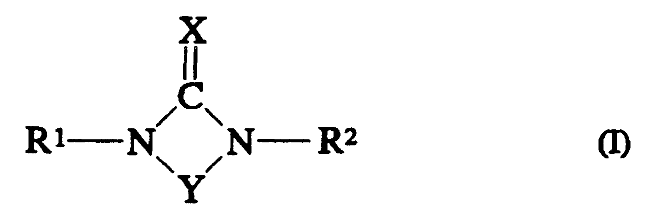 where
X is an oxygen or sulfur atom,
Y is a saturated or mono- or polyolefinically unsaturated carbon chain which has 2 to 8 carbon atoms and which is unsubstituted or mono- to trisubstituted by C₁-C₄-alkyl, C₁-C₄-alkoxy, C₂-C₄-acyl, C₂-C₄-acyloxy, phenoxy, C₂-C₈-dialkylamino, halogen, nitro and cyano and which may contain an ether, thioether, tertiary amino, keto, lactone, alkylsubstituted lactam, sulfone or diketo moiety, or is a cycloalkylene having 5 to 12 carbon atoms, heterocycloalkylene group having 4 to 11 carbon atoms, arylene group having 6 to 12 carbon atoms or hetarylene group having 3 to 11 carbon atoms, each of which is unsubstituted or mono- to trisubstituted by C₁-C₄-alkyl, C₁-C₄-alkoxy, C₂-C₄-acyl, C₂-C₄-acyloxy, phenoxy, C₂-C₈-dialkylamino, halogen, nitro and cyano,
and
R¹ and R², which may be identical or different, are C₁-C₂₀-alkyl, C₂-C₂₀-alkenyl or C₂-C₂₀-alkynyl, each of which is unsubstituted or mono- to trisubstituted by C₁-C₄-alkoxy, C₂-C₄-acyl, C₂-C₄-acyloxy, C₂-C₈-dialkylamino, halogen, nitro and/or cyano, or is C₂-C₂₀-acyl or is C₃-C₈-cycloalkyl, C₄-C₂₀-cycloalkylalkyl, C₃-C₈-heterocycloalkyl, C₄-C₂₀-heterocycloalkylalkyl, C₆-C₁₄-aryl or C₇-C₂₀-arylalkyl, each of which is unsubstituted or mono- to trisubstituted by C₁-C₄-alkyl, C₁-C₄-alkoxy, C₂-C₄-acyl, C₂-C₄-acyloxy, C₂-C₈-dialkylamino, halogen, nitro and/or cyano,
or
R¹ and R² are part of a mono- or polyolefinically unsaturated carbon chain having 2 to 8 carbon atoms, a cycloalkylene group having 5 to 12 carbon atoms, heterocycloalkylene group having 4 to 11 carbon atoms, arylene group having 6 to 12 carbon atoms or hetarylene group having 3 to 11 carbon atoms, which in each case connects the two nitrogen atoms,
and/or a compound of the formula II 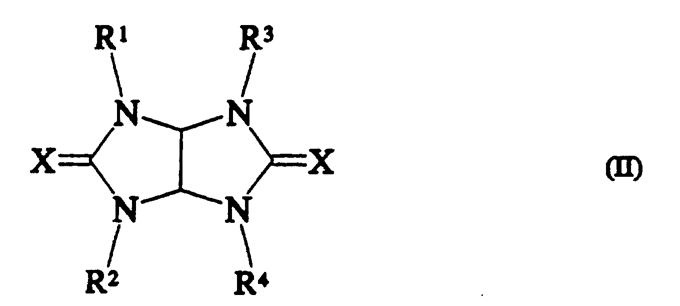 where X, R¹ and R² have the abovementioned meanings, and R³ and R⁴ have the meanings of R¹ and R²,
or
a compound of the formula III 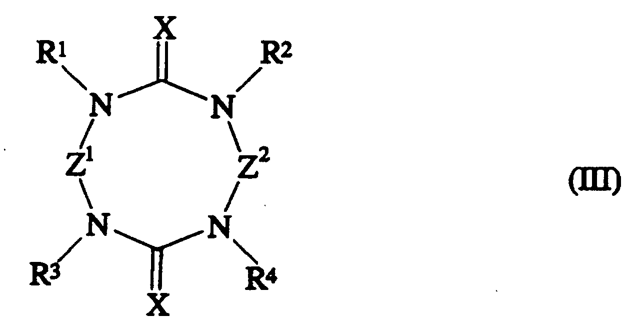 where X, R¹ to R⁴ have the abovementioned meanings, and Z¹, Z², which may be identical or different, are methylene, ethylene or vinylene groups which are unsubstituted or mono- to trisubstituted by C₁-C₄-alkyl, C₁-C₄-alkoxy, C₂-C₄-acyl, C₂-C₄-acyloxy, phenoxy, C₂-C₈-dialkylamino, halogen, nitro and/or cyano.

3. A process as claimed in claim 1 or 2, wherein N,N'-dimethylpropylene- or N,N'-dimethylethyleneurea is used as catalyst.

4. A process as claimed in any of claims 1 to 3, wherein C₁-C₈-alkanethiols, C₅-C₈-cycloalkanethiols, C₆- and C₁₂-arylthiols, C₇-C₁₂-aralkylthiols and/or C₃-C₁₂-heterocycloalkylthiols are reacted.

5. A process as claimed in any of claims 1 to 4, wherein from 0.02 to 1 mol% of the catalyst is employed per equivalent of thiol.

6. A process as claimed in any of claims 1 to 5, wherein the reaction is carried out at from 20 to 80°C under atmospheric pressure.

7. A process as claimed in any of claims 1 to 6, wherein the reaction is carried out in the melt of the thiol.

8. Process as claimed in any of claims 1 to 7, wherein the reaction is carried out in the presence of a solvent.

9. A process as claimed in claim 8, wherein the thiochloroformate derived from the thiol employed is used as solvent.

10. The use of cyclic ureas or thioureas as catalysts for preparing thiochloroformates by reacting thiols with phosgene in a process as claimed in claim 1.

## Revendications

1. Procédé de préparation de thiochloroformiates par réaction de thiols avec du phosgène en présence d'un catalyseur, **caractérisé en ce qu'**on utilise en tant que catalyseur une urée ou une thiourée cyclique, qui peut se présenter sous forme de ses sels pouvant être obtenus par réaction avec des acides halogènhydriques ou du phosgène.

2. Procédé selon la revendication 1, **caractérisé en ce qu'**on utilise en tant que catalyseur un composé de formule générale I 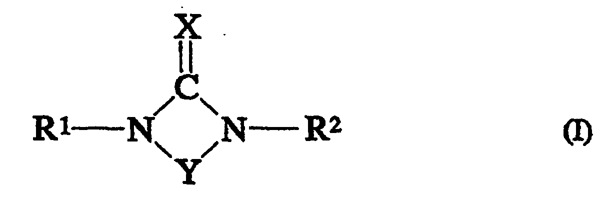 dans laquelle
X est un atome d'oxygène ou de soufre,
Y est une chaîne carbonée ayant de 2 à 8 atomes de carbone, saturée ou à une ou plusieurs insaturations oléfiniques, éventuellement une à trois fois substituée par des substituants alkyle en C₁-C₄, alcoxy en C₁-C₄, acyle en C₂-C₄, acyloxy en C₂-C₄, phénoxy, dialkylamino en C₂-C₈, halogéno, nitro et cyano, chaîne carbonée qui peut contenir un groupe éther, thioéther, amino tertiaire, céto, lactone, lactame alkylé, sulfone ou dicéto, ou encore un groupe cycloalkylène ayant de 5 à 12 atomes de carbone, éventuellement une à trois fois substitué par des substituants alkyle en C₁-C₄, alcoxy en C₁-C₄, acyle en C₂-C₄, acyloxy en C₂-C₄, phénoxy, dialkylamino en C₂-C₈, halogéno, nitro et cyano, un groupe hétérocycloalkylène ayant de 4 à 11 atomes de carbone, un groupe arylène ayant de 6 à 12 atomes de carbone ou un groupe hétéroarylène ayant de 3 à 11 atomes de carbone, et
R¹ et R², qui peuvent être identiques ou différents, représentent chacun un groupe alkyle en C₁-C₂₀, alcényle en C₂-C₂₀, alcynyle en C₂-C₂₀ ou acyle en C₂-C₂₀ éventuellement une à trois fois substitué par des substituants alcoxy en C₁-C₄, acyle en C₂-C₄, acyloxy en C₂-C₄, dialkylamino en C₂-C₈, halogéno, nitro et/ou cyano, ou un groupe cycloalkyle en C₃-C₈, cycloalkylalkyle en C₄-C₂₀, hétérocycloalkyle en C₃-C₈, hétérocycloalkyl-alkyle en C₄-C₂₀, aryle en C₆-C₁₄ ou arylalkyle en C₇-C₂₀ éventuellement une à trois substitué par des substituants alkyle en C₁-C₄, alcoxy en C₁-C₄, acyle en C₂-C₄, acyloxy en C₂-C₄, dialkylamino en C₂-C₈, halogéno, nitro et/ou cyano, ou bien
R¹ et R² sont chacun partie d'une chaîne carbonée ayant de 2 à 8 atomes de carbone, saturée ou ayant une ou plusieurs insaturations oléfiniques, reliant les deux atomes d'azote, un groupe cycloalkylène ayant de 5 à 12 atomes de carbone, un groupe hétérocycloalkylène ayant de 4 à 11 atomes de carbone, un groupe arylène ayant de 6 à 12 atomes de carbone ou un groupe hétéroarylène ayant de 3 à 11 atomes de carbone,
et/ou un composé de formule II 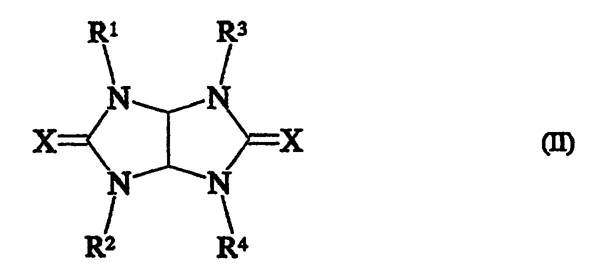 dans laquelle X, R¹ et R² ont les significations données ci-dessus, et R³ et R⁴ ont les significations de R¹ et R², ou bien
un composé de formule générale III 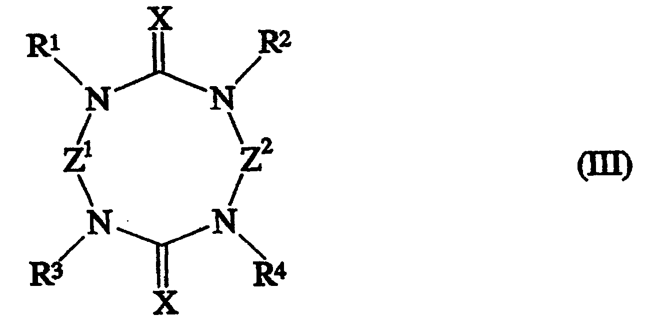 dans laquelle X, R¹ à R⁴ ont les significations données ci-dessus, et Z¹, Z², qui peuvent être identiques ou différents, représentent chacun un groupe méthylène, éthylène ou vinylidène éventuellement une à trois fois substitué par des substituants alkyle en C₁-C₄, alcoxy en C₁-C₄, acyle en C₂-C₄, acyloxy en C₂-C₄, dialkylamino en C₂-C₈, halogéno, nitro et/ou cyano.

3. Procédé selon la revendication 1 ou 2, **caractérisé en ce qu'**on utilise en tant que catalyseur de la N,N'-diméthylpropylène-urée ou de la N,N'-diméthyléthylène-urée.

4. Procédé selon l'une des revendications 1 à 3, **caractérisé en ce qu'**on fait réagir des alcanethiols en C₁-C₈, des cycloalcanethiols en C₅-C₈, des arylthiols en C₆ et en C₁₂, des aralkylthiols en C₇-C₁₂ et/ou des hétérocycloalkylthiols en C₃-C₁₂.

5. Procédé selon l'une des revendications 1 à 4, **caractérisé en ce qu'**on utilise par équivalent de thiol une quantité de 0,02 à 1 % en moles du catalyseur.

6. Procédé selon l'une des revendications 1 à 5, **caractérisé en ce qu'**on met en oeuvre la réaction à des températures de 20 à 80°C et sous la pression atmosphérique.

7. Procédé selon l'une des revendications 1 à 6, **caractérisé en ce qu'**on met en oeuvre la réaction dans un bain fondu de thiol.

8. Procédé selon l'une des revendications 1 à 7, **caractérisé en ce qu'**on met en oeuvre la réaction en présence d'un solvant.

9. Procédé selon la revendication 8, **caractérisé en ce qu'**on utilise en tant que solvant le thiochloroformiate qui dérive du thiol utilisé.

10. Utilisation d'urées ou de thiourées cycliques en tant que catalyseurs pour préparer des thiochloroformiates par réaction de thiols avec du phosgène dans un procédé selon la revendication 1.
